Europäisches Patentamt

European Patent Office

Office européen des brevets

(11) Publication number : **0 643 967 A2**

## (12) EUROPEAN PATENT APPLICATION

(21) Application number : 94306085.5

(22) Date of filing : 18.08.94

(51) Int. Cl.⁶ : **A61K 31/485,** A61K 31/235,
// (A61K31/485, 31:235)

(30) Priority : **24.08.93 GB 9317610**

(43) Date of publication of application :
**22.03.95 Bulletin 95/12**

(84) Designated Contracting States :
**DE GB**

(71) Applicant : **Euro-Celtique S.A.
122 Boulevard de la Petrusse
Luxemburg (LU)**

(72) Inventor : **Miller, Ronald Brown
Schutzenmattstrasse,
Basel, 4059 (CH)**

Inventor : **Leslie, Stewart Thomas
4 Babraham Road,
Cambridge (GB)**
Inventor : **Brown, Adrian
2A High Street,
Histon, Cambridge (GB)**
Inventor : **Malkowska, Sandra Therese
Antoinette
21 Broadway,
Wilburton,
Ely, Cambridge (GB)**
Inventor : **Prater, Derek Allan
28 Pearson Close,
Milton, Cambridge (GB)**

(74) Representative : **Lamb, John Baxter
MARKS & CLERK,
57-60 Lincoln's Inn Fields
London WC2A 3LS (GB)**

(54) **Pharmaceutical compositions containing analgesic, e.g. morphine, and a laxative, e.g. bisacodyl.**

(57) A pharmaceutical composition for oral administration containing, as active ingredients,
(i) an opioid analgesic or a pharmaceutically acceptable salt thereof ; and
(ii) a stimulant laxative.

EP 0 643 967 A2

This invention is concerned with improvements in and relating to pharmaceutical compositions. More particularly, the invention is concerned with pharmaceutical compositions comprising an opioid analgesic, for example morphine.

It is well known that opioid analgesics, as typified by morphine, have a constipatory effect when orally administered. In order to overcome this problem it has been proposed to administer opioid analgesics together with a laxative in the same unit dosage form. It is also known that laxatives such as bisacodyl can cause abdominal discomforts such as colic or cramp. It is recommended in order to avoid such gastric irritation that these tablets be enteric coated (see Martindale, The Extra Pharmacopoeia, 30th Edition, Academic Press, 1993).

It has now been found, in accordance with the present invention, that, unexpectedly, administration of an opioid analgesic such as morphine in conjunction with a stimulant laxative whilst reducing the constipatory effect of the opioid analgesic also reduces the unwanted side-effects of the laxative without recourse to enteric coating.

According to the invention, therefore, there is provided a pharmaceutical composition for oral administration containing, as active ingredients,

(i) an opioid analgesic or a pharmaceutically acceptable salt thereof, and

(ii) a stimulant laxative.

Typical stimulant laxatives for use in accordance with the invention include bisacodyl, casanthranol, cascara, danthron, senna and the sennosides, and sodium picosulphate. Of these, bisacodyl is generally preferred.

Typical opioid analgesics for use in accordance with the invention include morphine, hydromorphone, dihydrocodeine, and diamorphine. A particularly suitable pharmaceutically acceptable salt of morphine is morphine sulphate but, of course, other pharmaceutically acceptable salts may be used such as the hydrochloride, acetate or tartrate.

When using morphine and bisacodyl as active ingredients, the weight ratio of morphine (calculated as morphine sulphate) to bisacodyl in the compositions of the invention is suitably from 100:1 to 1:1, preferably 40:1 to 3:1. When, as is preferred as is discussed below, the composition of the invention is in dosage unit form, the dose of morphine (calculated as morphine sulphate) is suitably, for example, 30 mg/dosage unit form. As a result, the dose of bisacodyl is suitably 2.5 to 30 mg/dosage unit.

Where other stimulant laxatives are employed in place of bisacodyl, the amount of laxative employed will depend upon the nature of the laxative. Thus, typical daily dosages for various stimulant laxatives are, bisacodyl, 10-20 mg; casanthranol, 30-120 mg; cascara, 20-40 mg; danthron, 25-150 mg; the sennosides, 15-30 mg; and sodium picosulphate, 5-15 mg.

The pharmaceutical composition according to the present invention is useful in the treatment of pain associated with chronic medical conditions such as osteoarthritis, rheumatoid arthritis, ankylosing spondylitis, sero-negative arthropathies, cancer, etc. Compositions according to the invention are particularly effective in treating the pain of cancer.

Pharmaceutical compositions according to the present invention may be prepared by combining the active ingredients using conventional pharmaceutical techniques and are adapted for oral administration.

For oral administration, pharmaceutical compositions according to the invention may suitably take the form of tablets, capsules, granules, spheroids, powders, suspensions or liquid preparations. Preferably, the compositions are in dosage unit form, eg. in the form of tablets or capsules.

Tablets and capsules for oral administration may be prepared by conventional techniques with pharmaceutically acceptable excipients such as binding agents (for example pre-gelatinised maize starch or hydroxypropylmethyl cellulose); fillers (for example lactose, microcrystalline cellulose or calcium phosphate), lubricants (such as magnesium stearate, talc or silica), disintegrants, wetting agents, colourants and flavourings. The tablets may be coated according to methods well known in the art.

The analgesic and laxative components in medicaments according to the invention may be present in control release or normal release form. Preferably both are present in controlled release form.

Suitable materials for inclusion in a controlled release matrix include, for example:

(a) Hydrophilic or hydrophobic polymers, such as gums, cellulose esters, cellulose ethers, protein derived materials, nylon, acrylic resins, polyactic acid, polyvinylchloride, starches, polyvinylpyrrolidones, cellulose acetate phthalate. Of these polymers, cellulose ethers especially substituted cellulose ethers such as alkylcelluloses (such as ethylcellulose) $C_1$-$C_6$ hydroxyalkylcelluloses (such as hydroxypropyl-cellulose and especially hydroxyethyl cellulose) and acrylic resins (for example methacrylates such as methacrylic acid copolymers) are preferred. The controlled release matrix may conveniently contain between 1% and 80% (by weight) of the hydrophilic or hydrophobic polymer.

(b) Digestible, long chain ($C_8$-$C_{50}$, especially $C_8$-$C_{40}$), substituted or unsubstituted hydrocarbons, such as fatty acids, hydrogenated vegetable oils, such as Cutina (Trade Mark), fatty alcohols (such as lauryl, myristyl, stearyl, cetyl or preferably cetostearyl alcohol), glyceryl esters of fatty acids for example glyceryl

EP 0 643 967 A2

monostearate mineral oils and waxes (such as beeswax, glycowax, castor wax or carnauba wax). Hydrocarbons having a melting point of between 25°C and 90°C are preferred. Of these long chain hydrocarbon materials, fatty (aliphatic) alcohols are preferred. The matrix may contain up to 60% (by weight) of at least one digestible, long chain hydrocarbon.

(c) Polyalkylene glycols. The matrix may contain up to 60% (by weight) of at least one polyalkylene glycol.

A suitable matrix comprises one or more $C_{12}$-$C_{36}$, preferably $C_{14}$-$C_{22}$, aliphatic alcohols and/or one or more hydrogenated vegetable oils.

A particularly suitable matrix comprises one or more alkylcelluloses, one or more $C_{12-36}$, (preferably $C_{14}$-$C_{22}$) aliphatic alcohols and optionally one or more polyalkylene glycols.

Preferably the matrix contains between 0.5% and 60%, especially between 1% and 50% (by weight) of the cellulose ether.

The acrylic resin is preferably a methacylate such as methacrylic acid copolymer USNF Type A (Eudragit L, Trade Mark), Type B (Eudragit S, Trade Mark), Type C (Eudragit L 100-55, Trade Mark), Eudragit NE 30D, Eudragit E, Eudragit RL and Eudragit RS. Preferably the matrix contains between 0.5% and 60% by weight, particularly between 1% and 50% by weight of the acrylic resin.

In the absence of polyalkylene glycol, the matrix preferably contains between 1% and 40%, especially between 2% and 36% (by weight) of the aliphatic alcohol. When polyalkylene glycol is present in the oral dosage form, then the combined weight of the aliphatic alcohol and the polyalkylene glycol preferably constitutes between 2% and 40%, especially between 2% and 36% (by wt) of the matrix.

The polyalkylene glycol may be, for example, polypropylene glycol or, which is preferred, polyethylene glycol. The number average molecular weight of the at least one polyalkylene glycol is preferably between 200 and 15000 especially between 400 and 12000.

The medicament-containing controlled release matrix can readily be prepared by dispersing the active ingredient in the controlled release system using conventional pharmaceutical techniques such as wet granulation, dry blending, dry granulation or coprecipitation.

Controlled release spheroids may be prepared by spheronising the active ingredient together with a spheronising agent such as microcrystalline cellulose, and applying a release controlling coating comprising, for example, ethyl cellulose or an acrylic resin.

In order that the invention may be well understood the following Example is given by way of illustration only.

Example

Sustained release tablets were made up containing, per tablet:

| | |
|---|---|
| Morphine sulphate BP | 30.0 mg |
| Bisacodyl Ph.Eur. | 5.0 mg |
| Lactose (anydrous) U.S.N.F. | 70.0 mg |
| Hydroxyethylcellulose Ph.Eur. | 10.0 mg |
| Purified water Ph.Eur. | N.D. |
| Cetostearyl alcohol Ph.Eur. | 35.0 mg |
| Magnesium stearate Ph.Eur. | 2.0 mg |
| Purifed talc Ph. Eur. | 3.0 mg |
| | 155 mg |

When administered as a single dose, to healthy human volunters, no unwanted side effects from the laxative were noted.

**Claims**

1.   A pharmaceutical composition for oral administration containing, as active ingredients,

3

(i) an opioid analgesic or a pharmaceutically acceptable salt thereof; and
(ii) a stimulant laxative.

2. A composition as claimed in claim 1 in which the analgesic is morphine and the laxative is bisacodyl.

3. A composition as claimed in claim 1 substantially as hereinbefore described.